Europäisches Patentamt

(19) European Patent Office

Office européen des brevets

(11) Veröffentlichungsnummer: **0 193 538**
**B1**

(12) **EUROPÄISCHE PATENTSCHRIFT**

(45) Veröffentlichungstag der Patentschrifft:
21.03.90

(21) Anmeldenummer: 85903196.5

(22) Anmeldetag: 08.07.85

(86) Internationale Anmeldenummer:
PCT/CH 85/00108

(87) Internationale Veröffentlichungsnummer:
WO 86/01394 (13.03.86 Gazette 86/06)

(51) Int. Cl. 5: **A 61 F   2/34**

(54) KÜNSTLICHE HÜFTGELENKPFANNE MIT HYDROSTATISCHER KOPFLAGERUNG.

(30) Priorität: 28.08.84 CH 4121/84

(43) Veröffentlichungstag der Anmeldung:
10.09.86 Patentblatt 86/37

(45) Bekanntmachung des Hinweises auf die Patenterteilung:
21.03.90 Patentblatt 90/12

(54) Bennante Vertragsstaaten:
CH DE FR GB LI

(56) Entgegenhaltungen:
EP-A-0 053 794
CH-A-449 173
DE-A-2 229 812
DE-A-2 742 464
DE-A-2 822 585
FR-A-2 060 179
US-A-3 740 769
US-A-3 864 758

Physics in Medicine and Biology, Band 23, Nr. 2, März 1978, London (GB) A. Unsworth: "The effects of lubrication in hip joint prostheses", Seiten 253-268

(73) Patentinhaber: ROB. MATHYS CO.
CH-2544 Bettlach (CH)

(72) Erfinder: MATHYS, Robert
Chrüzliacher 11
CH-2544 Bettlach (CH)

(74) Vertreter: Lusuardi, Werther Giovanni
Dr. Lusuardi AG Kreuzbühlstrasse 8
CH-8008 Zürich (CH)

2

## Beschreibung

Die Erfindung betrifft eine künstliche Hüftgelenkpfanne gemäss dem Oberbegriff des Anspruchs 1.

Die bisher bekannten künstlichen Hüftgelenkpfannen, welche zum überwiegenden Teil aus HDPE (High Density Polyethylene) bestehen, zeigen bei zunehmender Belastung ein lineares Anwachsen des Reibungsmomentes zwischen Pfanne und Hüftgelenkkopf. Da bei normalem Gang eines Hüftgelenkprothesenträgers Spitzenbelastungen bis zum 7- bis 8fachen des Körpergewichtes auftreten können, treten bei konventionellen Pfannen erhebliche Reibungsmomente auf, welche zu einem übermässigen Verschleiss und zu einer Lockerung der Polyäthylenpfanne führen können.

Eine Anzahl von bekannten künstlichen Gelenkpfannen, beispielsweise gemäss der DE-A-2 229 812 und der DE-A-2 742 464, weisen Flüssigkeitskammern im Pfannenboden auf, deren Funktion es ist, unter dynamischen Verhältnissen, d. h. bei einer Relativbewegung von Kugelkopf und Pfanne, einen Flüssigkeitsfilm zur Herabsetzung der Reibung auszubilden. Um diesen dynamischen Schmiereffekt zu verstärken, sind bei diesen Pfannen nach dem Stand der Technik vorzugsweise Verbindungskanäle von der Flüssigkeitskammer im Pfannenboden nach aussen, bzw. zwischen mehreren Flüssigkeitskammern vorgesehen, damit Schmiermittel im dynamischen Verlauf angesaugt und in Umlauf gesetzt werden kann.

Bei der bekannten Pfanne gemäss der DE-A-2 742 464 wird für den Fall, dass keine Nut in der Pfanne vorgesehen ist, ein Schmiermittelkanal im Kugelkopf empfohlen. Ein Druckaufbau in der Flüssigkeitskammer soll somit bei diesen bekannten Pfannen auf jeden Fall ausgeschlossen werden. Damit können aber die oben erwähnten Nachteile nicht überwunden werden.

Der Erfindung liegt nun die Aufgabe zugrunde, eine künstliche Hüftgelenkpfanne zu schaffen, deren Reibungsmoment auch bei zunehmender Belastung nur geringfügig zunimmt und damit optimale tribologische Eigenschaften besitzt.

Die Erfindung löst die gestellte Aufgabe mit einer künstlichen Hüftgelenkpfanne, welche die Merkmale des Anspruchs 1 aufweist.

Dank dieser neuen Bauweise der erfindungsgemässen Hüftgelenkpfanne ergibt sich eine hydrostatische Lagerung des Hüftgelenkkopfes auf der mit Synovialflüssigkeit gefüllten und vom Gelenkkopf durch Deformation der elastischen Dichtungskante abgedichteten Flüssigkeitskammer. Mit zunehmender Belastung nimmt das Reibungsmoment nur geringfügig zu und strebt einem konstanten Wert zu, der bedeutend niedriger ist als bei konventionellen Hüftgelenkpfannen.

Bei einer aus einem elastischen Material bestehenden erfindungsgemässen Hüftpfanne wird die Flüssigkeitskammer durch eine Vertiefung im sphärischen Pfannenboden realisiert, so dass die Übergangszone zwischen sphärischer Innenfläche und Vertiefung die elastische Dichtungskante bildet.

Die erfindungsgemässe Hüftgelenkpfanne kann aber auch aus einem harten Material, beispielsweise aus einem Metall oder Keramik, bestehen mit einem elastischen Einsatz im Pfannenboden, der als Flüssigkeitskammer mit einer elastischen Dichtungskante ausgebildet ist.

Der Einsatz kann auch aus einem Dichtungsring aus elastischem Material bestehen.

Die Flüssigkeitskammer wird in der tatsächlichen Beanspruchungszone der Hüftgelenkkopfauflage angeordnet.

Der Durchmesser der Flüssigkeitskammer, bzw. der von ihr gebildeten latitudinalen Abdichtungszone, hängt von den übrigen Parametern des künstlichen Hüftgelenkes ab, weist aber vorteilhafterweise einen vom Hohlkugelzentrum aus gesehenen Öffnungswinkel von 10° - 120°, vorzugsweise von 30° - 90°, auf.

Die vorauf erwähnten Ausgestaltungen der Erfindung sind nebst praktischen Ausbildungsformen davon nachstehend an Hand der Zeichnungen näher erläutert. Es zeigt

Fig. 1    eine Ausführungsform der neuen Hüftgelenkpfanne in einem Schnitt durch den Beckenknochen;

Fig. 2    einen Schnitt in vergrösserter Darstellungsweise einer Hüftgelenkpfanne aus einem elastischen Material;

Fig. 3    einen Schnitt in vergrösserter Darstellungsweise einer Hüftgelenkpfanne aus einem starren Material.

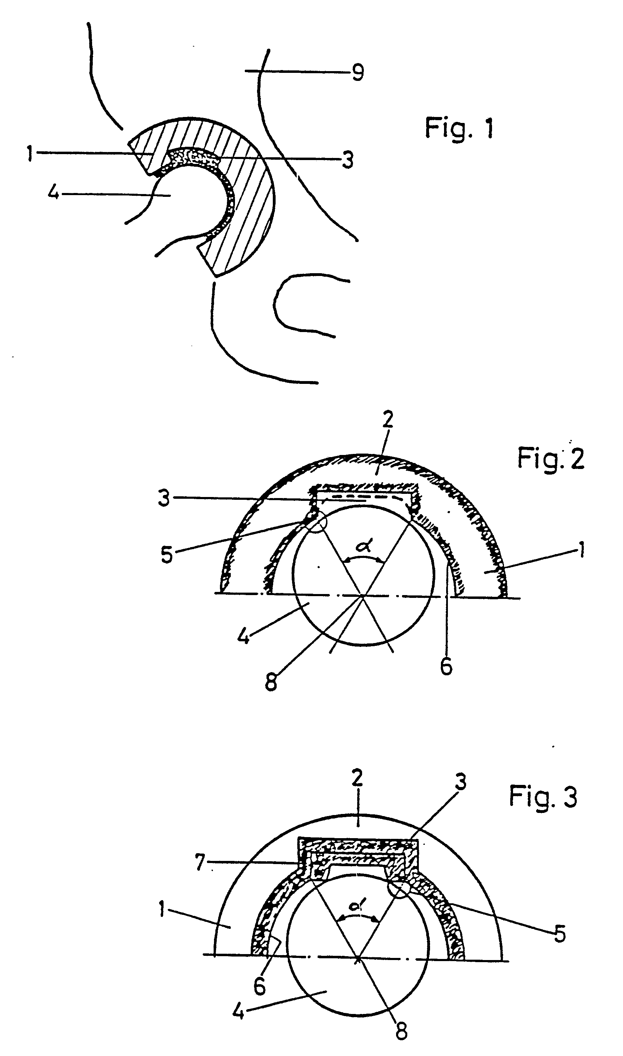

Die Hüftgelenkpfanne 1, welche aus einem üblichen Implantatwerkstoff bestehen kann, weist eine hohlkugelsegmentförmige Gestalt auf und besitzt eine im Pfannenboden 2 gelegene Flüssigkeitskammer 3. Wie aus Figur 1 ersichtlich, wird diese Flüssigkeitskammer 3 durch den eingreifenden künstlichen Hüftgelenkkopf 4 des Prothesenschaftes latitudinal, bzw. längs einer Zone der sphärischen Innenfläche 6, abgedichtet.

Bei den in den Figuren 2 und 3 dargestellten Hüftgelenkpfannen liegen die Flüssigkeitskammern entgegen dem im Anspruch 1 genannten Merkmal nicht in der Beanspruchungszone der Hüftgelenkkopfauflage. Diese Figuren sollen lediglich die Ausbildung der Dichtungskante zeigen.

Figur 2 zeigt eine Ausführungsform der Hüftgelenkpfanne 1 aus einem elastischen Kunststoffmaterial, beispielsweise aus Polyäthylen (HDPE), bei welcher der Rand der Flüssigkeitskammer 3, bzw. die Übergangszone zwischen sphärischer Innenfläche 6 und Flüssigkeitskammer 3 als elastische Dichtungskante 5 ausgebildet ist. Bei Belastung des künstlichen Hüftgelenkes deformiert sich die elastische Dichtungskante 5 zu einer entsprechenden Kugelzone, welche die mit Synovialflüssigkeit gefüllte Flüssigkeitskammer 3 abdichtet.

In Figur 3 ist eine Ausführungsform der Hüft-

In Figur 3 ist eine Ausführungsform der Hüftgelenkpfanne 1 aus einem starren Material, beispielsweise aus Aluminiumoxidkeramik, dargestellt. Die Abdichtung der Flüssigkeitskammer 3 erfolgt hier mittels eines elastischen Einsatzes 7 im starren Pfannenboden 2. Der in Figur 3 dargestellte elastische Einsatz 7 ist gleichzeitig als Auskleidung der Flüssigkeitskammer 3 ausgebildet. Es genügt jedoch für den angestrebten Abdichtungseffekt, den elastischen Einsatz 7 als Dichtungsring auszubilden.

In der Darstellung der Figur 1 ist die Flüssigkeitskammer 3 exzentrisch im Pfannenboden 2 in der tatsächlichen Beanspruchungszone der Hüftgelenkkopfauflage angeordnet um eine optimale Kräfteverteilung und Abdichtung zu erhalten.

Der Durchmesser der Flüssigkeitskammer 3 hängt vom jeweiligen Design und der Grösse der künstlichen Hüftgelenkpfanne ab und ist in der Darstellung der Figuren 2 und 3 durch einen vom Hohlkugelzentrum 8 aus gesehenen Öffnungswinkel α von 60° definiert.

**Patentansprüche**

1. Künstliche Hüftgelenkpfanne (1) hohlkugelsegmentförmiger Gestalt, mit einer im Pfannenboden (2) gelegenen Flüssigkeitskammer (3), dadurch gekennzeichnet, dass die Flüssigkeitskammer (3) durch elastische Deformation einer latitudinal sich erstreckenden, aus elastischem Material bestehenden Dichtungskante (5), welche durch die Übergangszone zwischen sphärischer Innenfläche (6) der Hüftgelenkpfanne (1) und Flüssigkeitskammer (3) gebildet wird, durch den eingreifenden künstlichen Hüftgelenkkopf (4) des Prothesenschaftes latitudinal derart abdichtbar ist, dass ein Druckaufbau in der Flüssigkeitskammer (3) ermöglicht wird, und dass die Flüssigkeitskammer (3) in der Beanspruchungszone der Hüftgelenkkopfauflage liegt.

2. Künstliche Hüftgelenkpfanne nach Anspruch 1, dadurch gekennzeichnet, dass die Hüftgelenkpfanne (1) aus einem im wesentlichen starren Material besteht mit einem im Pfannenboden (2) eingelassenen elastischen Einsatz (7), der als Flüssigkeitskammer (3) ausgebildet ist.

3. Künstliche Hüftgelenkpfanne nach Anspruch 1, gekennzeichnet durch einen latitudinalen Dichtungsring aus elastischem Material.

4. Künstliche Hüftgelenkpfanne nach einem der Ansprüche 1 bis 3, dadurch gekennzeichnet, dass der Durchmesser der latitudinalen Abdichtungszone (5), vom Hohlkugelzentrum (8) aus, einen Öffnungswinkel von 10° - 120°, vorzugsweise von 30° - 90° aufweist.

**Claims**

1. An artificial hip joint socket (1) having the form of a hollow spherical segment with a liquid chamber (3) being positioned in the bottom of the socket cavity (2), characterized in that the liquid chamber (3) - by means of elastic deformation of a latitudinally extending sealing edge (5) consisting of elastic material, which is formed by the transition zone between spherical inner surface (6) of the socket (1) and the liquid chamber (3) - is latitudinally sealable through the engaging ball head (4) of the femoral prosthesis shaft in such a way that a pressure build-up is made possible in the liquid chamber (3), and that the liquid chamber (3) is positioned at the stress zone of the femoral head support.

2. An artificial hip joint socket (1) according to claim 1, characterized in that the hip joint socket (1) consists of an essentially rigid material with an elastic insert (7) arranged in the bottom of the socket cavity (2), said insert (7) being designed as liquid chamber (3).

3. An artificial hip joint socket (1) according to claim 1, characterized in that it comprises a latitudinal sealing ring made from an elastic material.

4. An artificial hip joint socket (1) according to one of the claims 1 to 3, characterized in that the diameter of the latitudinal sealing zone (5) forms an aperture angle of 10° to 120°, preferably of 30° to 90°, with respect to the center of the hollow sphere (8).

**Revendications**

1. Cupule artificielle pour l'articulation de la hanche (1) en forme de segment sphérique creux à une base avec une chambre à liquide (3) située dans le fond de la cupule (2), caractérisée en ce que la chambre à liquide (3) - par déformation élastique d'une arête d'étanchéité (5) en matière élastique s'étendant latitudinalement, qui est formée par la zone de transition entre la surface sphérique intérieure (6) de la cupule pour l'articulation de la hanche (1) et la chambre à liquide (3) est rendue étanche latitudinalement par l'entrée en contact avec la tête artificielle (4) de la tige fémorale de la prothèse de hanche de telle manière qu'une augmentation de pression peut se produire dans la chambre à liquide (3) et en ce que la chambre à liquide (3) est située dans la zone de charge du support de la tête de la tige fémorale de la prothèse de hanche.

2. Cupule artificielle pour l'articulation de la hanche selon la revendication 1, caractérisée en ce que la cupule pour l'articulation de la hanche (1) consiste en un matériel essentiellement rigide avec une garniture élastique (7) située dans le fond de la cupule (2), ladite garniture (7) étant réalisée comme chambre à liquide (3).

3. Cupule artificielle pour l'articulation de la hanche selon la revendication 1, caractérisée en ce qu'elle comporte un anneau d'étanchéité latitudinal en matériel élastique.

4. Cupule artificielle pour l'articulation de la hanche selon une des revendications 1 à 3, caractérisée en ce que le diamètre de la zone d'étanchéité latitudinale (5) forme un angle d'ouverture entre 10° et 120°, préférablement entre 30° et 90°, par rapport au centre de la sphère creuse (8).

Fig. 1

Fig. 2

Fig. 3